# EUROPEAN PATENT SPECIFICATION

(11) **EP 2 620 168 B1**
(45) Date of publication and mention of the grant of the patent: **08.04.2020**
(21) Application number: 13152516.4
(22) Date of filing: 24.01.2013
(51) Int. Cl.: A61L 28/00, A61L 29/08, A61M 25/00

(54) **MULTI-LAYER CATHETER TUBES WITH ODOR BARRIER**
MEHRSCHICHTIGE KATHETERSCHLÄUCHE MIT GERUCHSBARRIERE
TUBES DE CATHÉTER MULTICOUCHE AVEC BARRIÈRE CONTRE LES ODEURS

(30) Priority: 27.01.2012 US 201213359968
(43) Date of publication of application: 31.07.2013
(73) Proprietor: Hollister Incorporated, Libertyville IL 60048 (US)
(72) Inventor: Holzbauer, Todd J., Hockessin, DE 19707 (US); Sadik, Adel, Fox River Grove, IL 60021 (US); Chang, Moh-Ching Oliver, Lake in the Hills, IL 60156 (US); Cisko, George, Jr., Spring Grove, IL 60081 (US)
(74) Representative: FRKelly

(56) References cited:
- WO-A1-2009/045874
- WO-A1-2011/056861
- WO-A2-2007/110080
- WO-A2-2008/052018
- US-A- 5 643 375
- US-A1- 2004 228 992

## Description

### Field of the Disclosure

This disclosure is generally directed to medical tubing and, more specifically, to multi-layered tubing having odor barrier properties suitable for use as catheter tubing for fecal drainage systems, and a combination odor barrier tube and odor barrier collection bag.

### Background

Examples of a catheter tube for a fecal drainage system are known from WO 2009/0458874 and US Patent No. 5,643,375. Catheter tubes for fecal drainage systems are designed to facilitate flow of fecal matter, with the fecal matter typically draining into a collection bag. A catheter tube can include a lubricious coating on an interior of the tube to facilitate movement of fecal matter. By providing a multi-layer catheter tube with at least one odor barrier layer, the catheter tube can reduce or eliminate exposure of the patient, caregivers, and other persons in the vicinity of the patient to unpleasant odors. Also, because it is desirable for rectal catheters to be retained in the rectal cavity for extended durations, it has been found beneficial to provide rectal catheters with a transsphincteric region that is collapsible in response to contraction of the patient's anal sphincter muscles, as described in US Patent Nos. 7,147,627 and 7,722,583 . Silicone is a preferred material for the transsphincteric region.

In providing a catheter tube extending between the transsphincteric region and a collection bag, not only it is desirable to prevent odor transmission, but it is also desirable to provide resistance to kinking of the catheter tube, because kinking can obstruct the flow of fecal matter therethrough. Fecal drainage systems are frequently employed in combination with enema, lavage, or other irrigation techniques to loosen stool in the rectum of a patient. As a result, water or other liquid is likely to travel through the catheter tube with fecal matter. While such liquid reduces the need to provide a lubricious surface on the interior of the catheter tube, it is desirable for the exterior of the catheter tube to be lubricious, as this facilitates milking the catheter tube to move waste material along the length of the catheter tube.

### Summary of the Disclosure

The present invention and the scope thereof is defined by the appended claims. The more generic description of the invention is provided for illustrative purposes only. Embodiments not falling under these claims are for reference purposes only.

The catheter tubing for a fecal drainage system is made of at least two layers of different materials. The layers are preferably co-extruded, or one layer may be extruded and one or more layers may be subsequently applied by extrusion over the first layer. Alternatively, the layers may be formed as a generally flat laminate sheet that is rolled into a tubular shape, then sealed along a seam, for example using sealing technology such as heat sealing, RF sealing, adhesive sealing, or ultrasonic welding. In order to increase the catheter tube's durability, a semi-rigid mesh or scrim material may be included as at least one of the layers of the flat laminate sheet that is rolled into a cylinder.

Another desirable feature of a catheter tube for a fecal drainage system is minimal wall thickness. The multi-layer odor barrier catheter tube of the present disclosure can be manufactured with a total wall thickness in the range of about 0.254 mm (10 mil) to about 1.524 mm (60 mil), and preferably with a total wall thickness of 0.635 mm (25 mil). The total wall thickness of 0.635 mm (25 mil) is preferable for all embodiments of the catheter tube described herein, regardless of the arrangement of catheter tube layers and the total number of layers.

The odor barrier layer or layers are made of nylon 669 or nylon 669 blended with reactive rubber. The odor barrier layer is sandwiched between two layers of thermoplastic elastomer (TPE). In another embodiment, a plurality of odor barrier layers may be alternately sandwiched between a plurality of layers of TPE. In yet another embodiment, the odor barrier layer is an intermediate layer of a plurality of layers of the catheter tube.

To prevent kinking of the catheter tube, which may adversely impact the catheter tube's function, a low compression set thermoplastic elastomer is preferably used for each of catheter tube's TPE layers. The inner TPE layer, which is thicker than the one or more outer TPE layers and which makes up the bulk of the overall thickness of the catheter tube, preferably has a thickness in a range of 0.304 mm to 0.608 mm (12 mil to 24 mil), and most preferably has a thickness of 0.456 mm (18 mil). The outer TPE layer preferably has a thickness in the range of 0.127 mm to 0.203 mm (5 mil to 8 mil), and most preferably has a thickness of 0.152 mm (6 mil). The outer TPE layer may be formulated so as to exhibit high lubricity, i.e. a low coefficient of friction. High lubricity of the outer TPE layer facilitates relative movement of a latex- or non-latex-gloved hand along the exterior of the catheter tube when milking contents of the catheter tube toward a collection bag in fluid communication with the interior of the catheter tube.

While lubricity of the inner TPE layer may also be desirable to promote or facilitate flow of fecal matter through the catheter tube, it is found that there is typically sufficient liquid content with the waste matter draining through the catheter tube such that the inner TPE layer need not exhibit as high a lubricity as the outer TPE layer.

The odor barrier layer of the catheter tube of this embodiment preferably has a thickness of 0.076 mm (3 mil) or less, most preferably 0.025 mm (1 mil), and the thickness of the odor barrier is preferably less than 30% of the total tube wall thickness.

In an embodiment, not being part of the present invention, one or more layers of TPE may be sandwiched between two or more layers of odor barrier material such as nylon PA 669 or nylon PA 669 blended with a reactive rubber. In another embodiment, a TPE layer is an intermediate layer of a plurality of layers of the catheter tube.

In this embodiment, the inner TPE layer, which also makes up the bulk of the overall thickness of the catheter tube, preferably has a thickness in a range of 0.254 mm to 1.524 mm (10 mil to 60 mil), and most preferably has a thickness of 0.604 mm (24 mil). Each of the outer odor barrier layers preferably has a thickness in the range of 0.006 mm to 0.038 mm (0.25 mil to 1.5 mil), and most preferably has a thickness of 0.013 mm (0.5 mil), such that the overall thickness of the catheter tube is, most preferably, 0.635 mm (25 mil). While lubricity of the inner nylon layer may be desirable to promote or facilitate flow of fecal matter through the catheter tube, as before, there may be sufficient liquid content with the waste matter draining through the catheter tube of this embodiment such that the inner nylon layer need not exhibit as high a lubricity as the outer nylon layer.

A beneficial aspect of the catheter tube of embodiments of this disclosure is the ability of the catheter tube to block unpleasant odors. However, if the collection bag into which the catheter tube drains lacks odor barrier properties, unpleasant odors can escape the collection bag, thereby negating the odor barrier benefits achieved by the odor barrier catheter tube of this disclosure. It is therefore preferable that the odor barrier tube to drain into a collection bag that itself has odor barrier walls.

### Brief Description Of The Several Views Of The Drawing

FIG. 1 is a perspective view of a first embodiment of a multi-layer odor barrier catheter tube of the present disclosure;
FIG. 2 is an axial cross-sectional view, taken along lines 2-2 of FIG. 1;
FIG. 3 is an axial cross-sectional view, similar to FIG. 2, of a second embodiment of a multi-layer odor barrier catheter tube of the present disclosure, wherein tie layers are provided between the first and second layers, and between the second and third layers;
FIG. 4 is a perspective view of a heat-laminated film employed in a method of manufacture of a multi-layer odor barrier catheter tube of the present disclosure;
FIG. 5 is a perspective view of the heat-laminated film of FIG. 4, rolled into a cylindrical shape and sealed along a seam;
FIG. 6 is a perspective view of a heat-laminated film similar to that of FIG. 4, and including a flexible scrim layer;
FIG. 7 is a perspective view of the heat-laminated film of FIG. 6, rolled into a cylindrical shape and sealed along a seam;
FIG. 8 is a front view of a combination of a multi-layer odor barrier catheter tube of the present disclosure in combination with a drainable collection bag having odor barrier walls;
FIG. 9 is a front perspective view of a combination of a multi-layer odor barrier catheter tube of the present disclosure in combination with a closed, single-use collection bag having odor barrier walls;
FIG. 10 is a perspective view of a third embodiment of a multi-layer odor barrier catheter tube of the present disclosure, including an outermost ethylene methyl acrylate (EMA) film surrounding an outer thermoplastic elastomer layer, the EMA film serving as a silicone bonding substrate;
FIG. 11 is a cross-sectional view, taken along lines 11-11 of FIG. 10;
FIG. 12 is a perspective view of another embodiment of a multi-layer odor barrier catheter tube, which is not part of the present invention, wherein the interior and exterior layers comprise an odor barrier material; and
FIG. 13 is a cross-sectional view, taken along lines 13-13 of FIG. 12.

### Detailed Description of the Preferred Embodiments

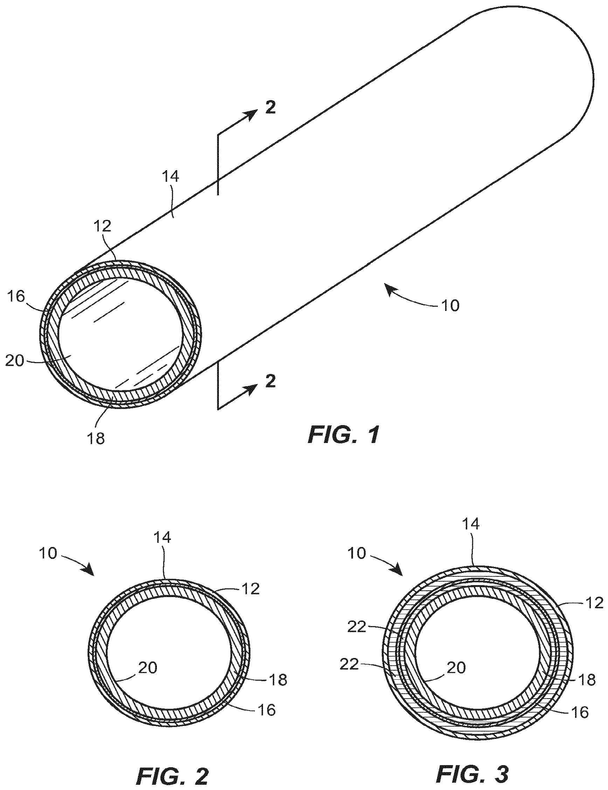

With reference to FIGS. 1-2, in a first embodiment of a multi-layer odor barrier catheter tube 10 of the present disclosure, the catheter tube 10 is connectable at a first end to a patient-proximal internal section, and/or a trans-anal section of a rectal catheter (not shown). The catheter tube 10 is also connectable at a second end to a waste collection bag or to a disposal receptacle (e.g., a bedpan or toilet (not shown)). At least one layer of the multi-layer catheter tube 10 is constructed of a material resistant to transmission of fecal and flatus gasses. The material is nylon PA 669. EMS-Grivory's GRILON® BM 13 SBG is a suitable low-modulus nylon, with its modulus of approximately 296 Kg/mm² (43 ksi) compared to typical nylon PA6 with a modulus of about 689 kg/mm² (100 ksi). Another suitable material is nylon PA669 blended with reactive rubber to reduce the modulus and set of the blend. For example, nylon PA669 could be blended with Kraton® FG1924, LOTADER® 4720, or DuPont Fusabond® 493. A suitable blend would be, for example, 75% EMS-Grivory GRILON® BM 13 SBG compounded with 25% LOTADER® 4720. In addition, one or more lubricants may be added to the blend. These lubricants may include, for example, an amide wax (e.g., erucamide, oleamide, stearyl erucamide), an ester wax (e.g., esters of montanic acids), or silicon oil preferably used at 0.05% to 0.5% loading to generate a low surface coefficient of friction.

A first layer 12 of the catheter tube 10 defines the external surface 14 of the catheter tube 10. The first layer 12 is preferably comprised of one or more materials that possess a relatively low coefficient of friction, most preferably less than 0.5, so as to reduce drag of the catheter tube 10 against a patient's skin and against any items surrounding the patient, such as a hospital gown, bed sheets, or a chair. The low coefficient of friction of the external surface 14 of the catheter tube 10 also facilitates "milking" the fecal matter down the length of the catheter tube 10.

The material or materials defining the first layer 12 of the catheter tube 10 include a thermoplastic elastomer (TPE), preferably having a thickness in the range of 0.101 mm to 0.203 mm (4 mil to 8 mil), and most preferably a thickness of 0.152 mm (6 mil). TPE exhibits adequate lubricity, particularly TPE having a Shore A hardness of approximately 60. The external surface 14 of the first layer 12 is preferably a surface that will receive inks such as permanent or semi-permanent markers and retain such markings thereon without smudging or wiping off. This will facilitate receiving instructions, patient data, collection bag change data, dates of indwell or intended removal of the catheter tube, and other such indicia.

The intermediate layer 16 of the catheter tube 10 of the present invention is an odor barrier film layer, which is nylon 669.

An inner layer 18 of the multi-layer catheter tube 10 of an embodiment of the present disclosure preferably comprises TPE having a thickness in a range of 0.304 mm to 0.608 mm (12 mil to 24 mil). The inner layer 18 most preferably has a thickness of 0.457 mm (18 mil). The inner layer 18 is preferably the thickest layer of the catheter tube 10 and is formulated for low compression-set, with high elasticity, as this is found to render the catheter tube 10 more resistant to developing kinks, such as when folded in packaging material.

Depending on the compatibility of the materials of adjacent layers of the multi-layered catheter tube 10, as shown in FIG. 3, a tie layer 22, such as DuPont Byne®, may be employed between adjacent layers to improve adhesion of the adjacent layers to one another. However, in a preferred embodiment of the present disclosure, the materials of adjacent layers of the multi-layer catheter tube are compatible and do not require a tie layer be employed between adjacent layers to improve adhesion of the adjacent layers to one another.

The multi-layer catheter tube 10 of the first embodiment of the present disclosure may be manufactured by co-extrusion. In order to maximize tube softness and flexibility, it is desirable that a catheter tube 10 of some embodiments of the present disclosure include a single, thin odor barrier layer, preferably 0.025 mm (1 mil). In embodiments of the catheter tube 10 with more than one odor barrier layer, the odor barrier layers preferably have a thickness of 0.006 mm to 0.038 mm (0.25 mil to 1.5 mil).

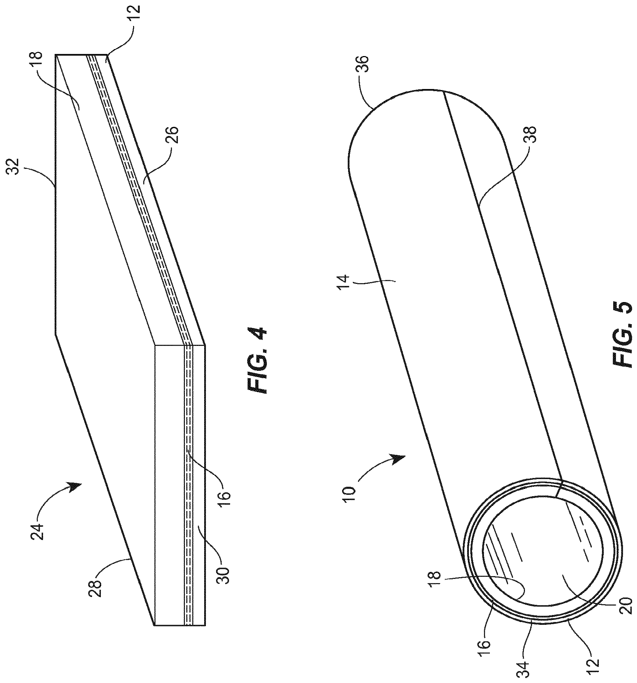

Turning to FIGS. 4-5, as an alternative to co-extrusion, the layers of the catheter tube 10 may be formed into a flat or substantially flat heat-laminated film 24 and then rolled into a cylinder. For example, in one embodiment of the present disclosure as illustrated in FIGS. 4-5, two parallel edges 26, 28 are rolled toward one another, brought into register with one another, and sealed to one another, such as by heat sealing, RF sealing, adhesive sealing, or ultrasonic welding, to form a cylinder, with the first layer 12 of the film 24 defining an external surface 14 of the cylinder and the third layer 18 defining an internal surface 20 of the cylinder. A leading end 30 and a trailing end 32 of the heat-laminated film 24 are left open, forming first and second ends 34, 36 of the catheter tube 10. Optionally, tie layers (represented by broken lines in FIG. 4) may be provided to enhance bonding between the inner layer 18 and intermediate odor barrier layer 16, and/or between the intermediate layer 16 and the outer layer 12. The first, second, and intermediate layers 12, 18, 16 of the catheter tube 10 are preferably clear or translucent. The sealed edge or seam 38 may be visible and can advantageously provide a medical caregiver with a visible indicator of any kinking or twisting of the catheter tube.

The total cumulative wall thickness of the multi-layer catheter tube 10 is preferably in the range of about 0.254 mm to about 1.016 mm (10 mil to about 40 mil), and more preferably in a range of about 0.635 mm to about 0.889 mm (25 mil to about 35 mil), with the thickness of the odor barrier layer preferably making up less than about 30% of the total wall thickness of the catheter tube 10.

Adjustments may be made to process conditions under which the layers of the multi-layer catheter tube of the present disclosure are co-extruded or heat laminated to reduce the coefficient of friction of one or more of the layers.

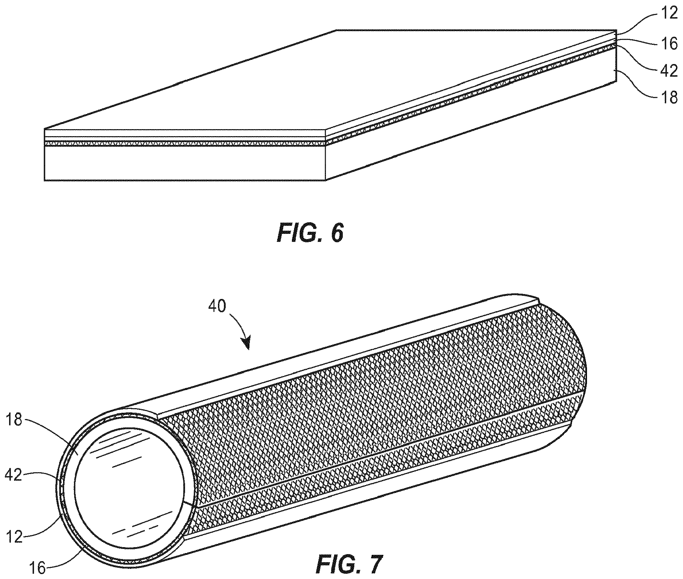

In another embodiment, shown in FIGS. 6-7, in order to provide reinforcement and avoid kinking or twisting of a catheter tube 40, a mesh or flexible scrim layer 42 may be included as an additional layer intermediate the first layer 12 and intermediate layer 18 of the multi-layer catheter tube 40. The scrim layer 42, if provided, can serve to provide the catheter tube 40 with shape memory, permitting the catheter tube 40 to collapse and recover to its cylindrical shape without any permanent deformation. Materials other than a scrim layer 42 may be used instead of or in addition to the scrim layer 42 to enhance structural integrity of the catheter tube 40, such as polymeric materials.

The material(s) forming the internal and/or external surfaces of the multi-layer catheter tube of the present disclosure preferably facilitate attachment and assembly of the catheter tube to peripheral components of fecal drainage and management systems, such as the Bowel Management System available from Hollister Incorporated of Libertyville, Illinois. As such systems are intended for long-duration use, on the order of about twenty-nine days, it is advantageous to employ materials that will easily form a reliable bond, by adhesive and/or heat, between the catheter tube 10 and the peripheral components, such as internal or external silicone balloons, catheter connections (e.g., a collection bag or a catheter tube extension), plastic or metal ports (e.g., ports for providing endoscope access or for sampling fecal matter directly from the catheter tube) for the entire duration of use of the catheter tube 10.

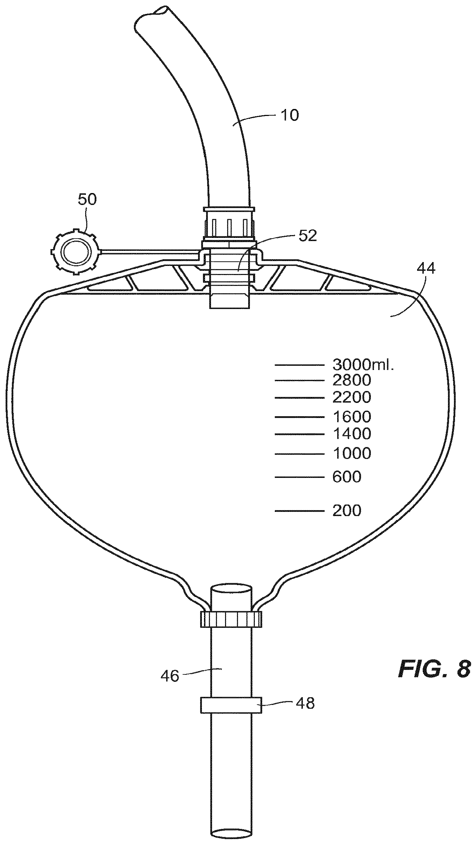

As noted above, the advantages achieved by the odor barrier properties of the catheter tubes of the present disclosure would be negated, or significantly diminished, if fecal or flatus gasses could be transmitted through one or more walls of a collection bag 44 (see FIG. 8) to which the catheter tube 10 is connected. It is therefore desirable to use the multi-layer odor barrier catheter tube 10 in combination with a collection bag 44 having odor barrier walls. For example, each of the walls of the collection bag may include a barrier layer film such as the one disclosed in U.S. Patent No. 7,270,860.

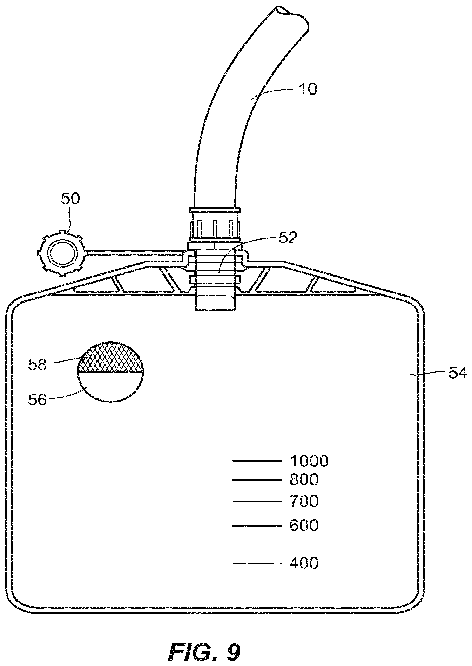

Turning to FIG. 8, the odor barrier collection bag may be a drainable collection bag 44, having a drainage tube 46 with a drainage tube stopper 48 and a cap 50 for capping a catheter tube connection port 52. Alternately, as illustrated in FIG. 9, the odor barrier collection bag may be a so-called "closed" collection bag 54. A closed collection bag 54 is intended for single use, and preferably includes an integral vent 56 with a deodorizing filter 58.

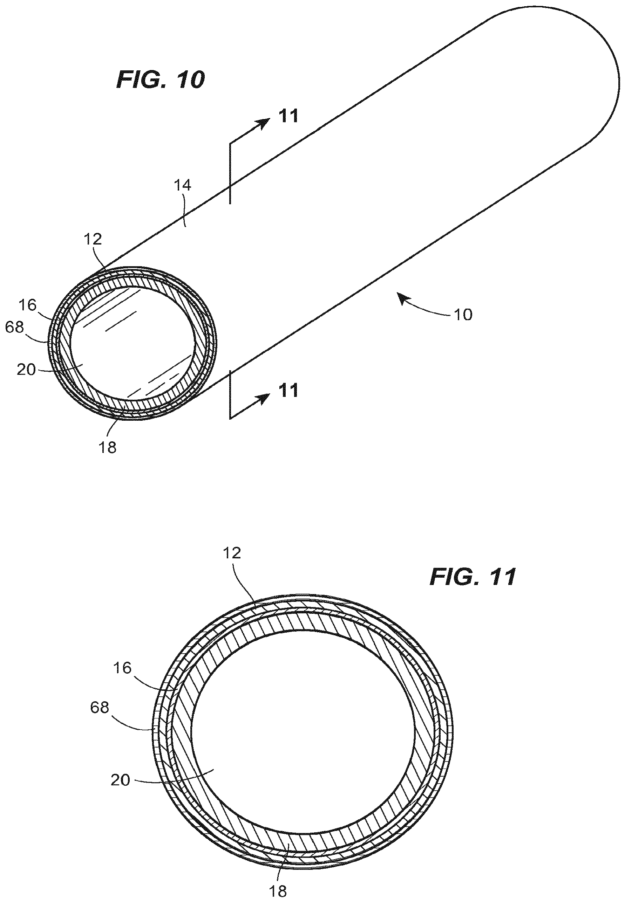

Turning to FIGS. 10 and 11, an alternate embodiment of a catheter tube is illustrated in which the outer TPE layer 12 of the catheter tube 10 has an ethylene methyl acrylate (EMA) film 68 bonded, such as by heat sealing, to at least the portion of the exterior thereof that is received in the silicone transsphincteric region 60. EMA film 68 bonds to silicone more readily than TPE. EMA film also exhibits high lubricity, thereby eliminating the need to formulate the outer TPE layer 12 in a manner that increases that layer's lubricity, or eliminating the need to add a lubricious coating, such as Parylene, to the exterior of the catheter tube 10.

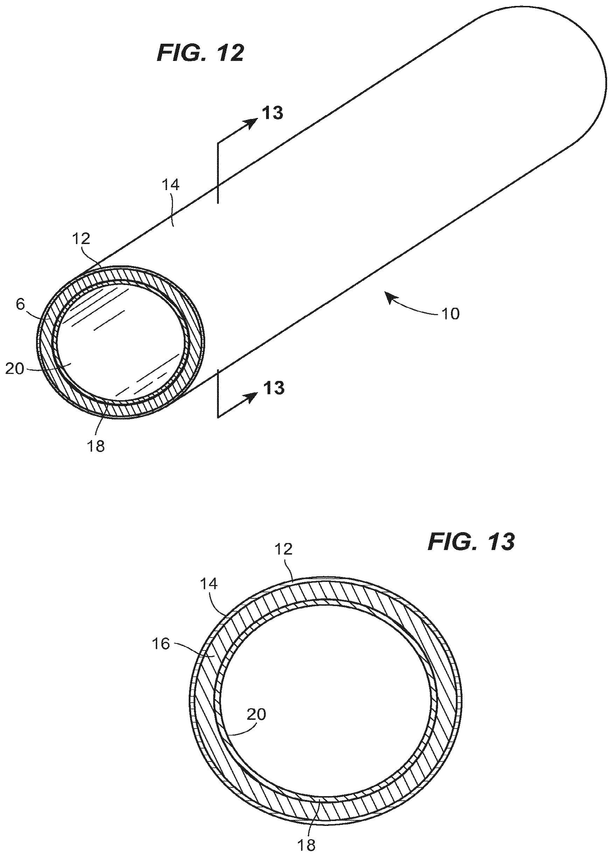

Turning to FIG. 12, in another embodiment not being part of the present invention, an intermediate layer 16 comprising TPE may be sandwiched between a first layer 12 and an inner layer 18, both comprising an odor barrier material such as nylon PA 669 or nylon PA 669 blended with a reactive rubber. A beneficial aspect of this embodiment is that by sandwiching the intermediate layer 16 of TPE between the odor barrier layers 12, 13, difficulties of bonding TPE to silicone device components to which the multi-layer tubing 10 is attached may be avoided due to the compatibility of the nylon odor barrier material and silicone.

The TPE layer 16, which makes up the bulk of the overall thickness of the catheter tube 10 of this embodiment, preferably has a thickness in a range of 0.508 mm to 1.016 mm (20 mil to 40 mil), and most preferably has a thickness of 0.609 mm (24 mil). Each of the outer odor barrier layers 12, 18 preferably has a thickness in the range of 0.006 mm to 0.038 mm (0.25 mil to 1.5 mil), and most preferably has a thickness of 0.0012 mm (0.5 mil), such that the overall thickness of the catheter tube is, most preferably, 0.635 mm (25 mil). Furthermore, while lubricity of the inner nylon layer 18 may be desirable to promote or facilitate flow of fecal matter through the catheter tube 10, there may be sufficient liquid content with the waste matter draining through the catheter tube 10 of this embodiment such that the inner nylon layer 18 need not exhibit as high a lubricity as the outer nylon layer 12. In another embodiment of the present disclosure, the inner layer may be hydrophobic.

In each of the foregoing embodiments of the present disclosure, the TPE material may include or may be substituted by thermoplastic polyurethane (TPU), styrene-butadiene copolymer (SBC), thermoplastic vulcanisate (TPV), ethylene propylene diene monomer (EPDM), or any other suitable copolymer or blend.

## Claims

1. A catheter tube (10) for a fecal drainage system comprising:
a first layer (12) including a thermoplastic elastomer;
a second layer (18) including a thermoplastic elastomer; and
a film layer (16) intermediate the first layer (12) and the second layer (18), the film layer including an odor barrier material, which is nylon 669.

2. The catheter tube of claim 1, wherein the first layer (12) defines an internal surface of the catheter tube and is the thickest layer of the catheter tube.

3. The catheter tube of claim 2, wherein the film (16) layer intermediate the first layer (12) and the second layer (18) is the thinnest layer of the catheter tube.

4. The catheter tube of claim 1, wherein the film layer (16) intermediate the first layer and the second layer is nylon 669 blended with a reactive rubber.

5. The catheter tube of claim 4, wherein the nylon 669 blended with a reactive rubber further comprises one or more lubricants.

6. The catheter tube of claim 1, further comprising a fourth layer bonded about at least a portion of an exterior of the second layer (18), the fourth layer including an ethylene methyl acrylate (EMA) film.

7. A catheter tube for a fecal drainage system according to claim 1, comprising
at least one of the first and second layers including the thermoplastic elastomer material, which includes ethylene methyl acrylate film (EMA), thermoplastic polyurethane (TPU), styrene-butadiene copolymer (SBC), thermoplastic vulcanisate (TPV), or ethylene propylene diene monomer (EPDM).

## Patentansprüche

1. Katheterschlauch (10) für ein Stuhlentleerungssystem, umfassend:
eine erste Schicht (12), die ein thermoplastisches Elastomer beinhaltet;
eine zweite Schicht (18), die ein thermoplastisches Elastomer beinhaltet; und
eine Folienschicht (16), die zwischen der ersten Schicht (12) und der zweiten Schicht (18) liegt, wobei die Folienschicht ein Geruchsbarrierematerial beinhaltet, welches Nylon 669 ist.

2. Katheterschlauch nach Anspruch 1, wobei die erste Schicht (12) eine Innenfläche des Katheterschlauchs definiert und die dickste Schicht des Katheterschlauchs ist.

3. Katheterschlauch nach Anspruch 2, wobei die Folienschicht (16), die zwischen der ersten Schicht (12) und der zweiten Schicht (18) liegt, die dünnste Schicht des Katheterschlauchs ist.

4. Katheterschlauch nach Anspruch 1, wobei die Folienschicht (16), die zwischen der ersten Schicht und der zweiten Schicht liegt, Nylon 669, das mit einem Reaktivkautschuk gemischt ist, ist.

5. Katheterschlauch nach Anspruch 4, wobei das Nylon 669, das mit einem Reaktivkautschuk gemischt ist, ferner ein oder mehrere Schmiermittel umfasst.

6. Katheterschlauch nach Anspruch 1, ferner umfassend eine vierte Schicht, die um mindestens einen Abschnitt eines Äußeren der zweiten Schicht (18) geklebt ist, wobei die vierte Schicht eine Ethylenmethylacrylat-(EMA)folie beinhaltet.

7. Katheterschlauch für ein Stuhlentleerungssystem nach Anspruch 1, umfassend
mindestens eine aus der ersten und der zweiten Schicht, die das thermoplastische Elastomermaterial beinhalten, das eine Ethylenmethylacrylatfolie (EMA), ein thermoplastisches Polyurethan (TPU), ein Styrolbutadiencopolymer (SBC), ein thermoplastisches Vulkanisat (TPV) oder ein Ethylen-Propylen-Dien-Monomer (EPDM) beinhaltet.

## Revendications

1. Tube de cathéter (10) pour un système de drainage fécal comprenant :
une première couche (12) comportant un élastomère thermoplastique ;
une seconde couche (18) comportant un élastomère thermoplastique ; et
une couche de film (16) entre la première couche (12) et la seconde couche (18), la couche de film comportant un matériau formant barrière aux odeurs, qui est le nylon 669.

2. Tube de cathéter selon la revendication 1, dans lequel la première couche (12) définit une surface interne du tube de cathéter et est la couche la plus épaisse du tube de cathéter.

3. Tube de cathéter selon la revendication 2, dans lequel la couche de film (16) entre la première couche (12) et la seconde couche (18) est la couche la plus fine du tube de cathéter.

4. Tube de cathéter selon la revendication 1, dans lequel la couche de film (16) entre la première couche et la seconde couche est le nylon 669 mélangé à un caoutchouc réactif.

5. Tube de cathéter selon la revendication 4, dans lequel le nylon 669 mélangé avec un caoutchouc réactif comprend en outre un ou plusieurs lubrifiants.

6. Tube de cathéter selon la revendication 1, comprenant en outre une quatrième couche liée autour d'au moins une partie d'un extérieur de la seconde couche (18), la quatrième couche comportant un film en éthylène-acrylate de méthyle (EMA).

7. Tube de cathéter pour un système de drainage fécal selon la revendication 1, comprenant
au moins l'une des première et seconde couches comportant le matériau élastomère thermoplastique, qui comporte un film d'éthylène-acrylate de méthyle (EMA), du polyuréthane thermoplastique (TPU), un copolymère styrène-butadiène (SBC), un vulcanisat thermoplastique (TPV), ou un monomère éthylène propylène diène (EPDM).
